# EUROPEAN PATENT SPECIFICATION

(11) **EP 1 004 594 B1**
(45) Date of publication and mention of the grant of the patent: **06.08.2003**
(21) Application number: 99122978.2
(22) Date of filing: 19.11.1999
(51) Int. Cl.: C07J 9/00, A23L 1/30, A23D 9/013

(54) **Use of Phytosterol and/or phytostanol esters**
Verwendung von Phytosterol und/oder Phytostanolestern
Utilisation d'esters de phytostérol et/ou phytostanol

(30) Priority: 26.11.1998 EP 98122412; 29.09.1999 EP 99119337
(43) Date of publication of application: 31.05.2000
(73) Proprietor: F. HOFFMANN-LA ROCHE AG, 4070 Basel (CH)
(72) Inventor: Burdick, David Carl, 4102 Binningen (CH); Moine, Gérard, 68400 Riedisheim (FR); Raederstorff, Daniel, 68350 Brunstatt (FR); Weber, Peter, 79429 Malsburg-Marzell (DE)
(74) Representative: Kellenberger, Marcus Dr.

(56) References cited:
- WO-A-00/04887
- WO-A-98/06405
- US-A- 4 588 717
- US-A- 5 593 691
- SHIMADA, YUJI; HIROTA, YOSHINORI; BABA, TAKASHI; SUGIHARA, AKIO; MORIYAMA, SHIGERU; TOMINAGA, YOSHIO; TERAI, TADAMASA.: "Enzymatic Synthesis of Steryl Esters of Polyunsaturated Fatty Acids" JOURNAL OF THE AMERICAN OIL CHEMISTS SOCIETY , vol. 76, no. 6, June 1999 (1999-06), pages 713-716, XP002132268

## Description

The present invention relates to the use of polyunsaturated fatty acid esters of phytosterols and/or phytostanols.

Phytosterols are plant sterols found, for example, in small amounts in vegetable oils such as corn, bean or other plant oils, where they occur as the free sterols, fatty acid esters,and glycosides. Phytosterols are structurally similar to cholesterol, the main differences occuring in carbon skeleton of their side chains. A number of different phytosterol structures are found in nature. The most common ones are campesterol, beta-sitosterol and stigmasterol. Reduction of phytosterols yields saturated phytosterols, called phytostanols, such as campestanol or sitostanol, which also occur naturally in small amounts. A normal human diet typically leads to ingestion of less than one half gram a day of such substances in various forms.

It is known that ingestion of phytosterols and/or phytostanols in defined amounts, for example of several grams a day or more, can reduce blood serum cholesterol levels. It is assumed that free phytosterols and phytostanols inhibit the uptake of dietary and biliary cholesterol through displacement of cholesterol. However, generally only modest reductions of serum cholesterol levels have been observed by adding free phytosterols or phytostanols to the diet.

Arteriosclerosis is a leading cause of death in many parts of the Western world. It has been shown that low density lipoprotein (LDL) cholesterol is directly associated with the development of cardiovascular disease, whereas high density lipoprotein (HDL) cholesterol has an inverse relationship with cardiovascular disease development. People with combined hyperlipidemia run even higher risks of heart disease. Elevated blood serum levels of cholesterol and elevated levels of triglycerides are generally accepted both as causes and as indicators of the progression of cardiovascular disease. Thus lowering cholesterol and lowering triglycerides are both seen as desirable goals and major strategies for intervention. Many methods have been proposed to lower serum cholesterol, among them use of certain pharmaceutical agents and the ingestion of phytosterols in various forms. Likewise, many methods have been proposed to lower serum triglycerides, among them ingestion of polyunsaturated fatty acids (PUFAs) in various forms.

Physical properties are especially important in food applications. Properties of ingredients allow and limit the forms into which the products can be delivered e.g. in oils or butters. Further, properties such as solubility and melting point can affect acceptability of a food product by changing texture, mouth feel or taste in complicated, unpredictable ways. One problem with the use of free phytosterols has been their crystalline nature and limited solubility in oils. Generally, large amounts have been used to achieve effect on cholesterol levels but with resultant physical problems. Thus other forms have been sought.

WO 96/38047 discloses a fat-based food product comprising natural fat components which have a blood cholesterol lowering effect and wherein at least one component of tocotrienol, oryzanol and phytosterol is present physically mixed preferably with at least one component of PUFA-triglycerides. The phytosterols present are mainly in free phytosterol form in low, defined concentrations, and relatively insoluble. The resultant products are semi solids. Much higher amounts proportionally of the PUFA triglycerides to phytosterols are used. Effects of the mixtures on triglyceride levels are not described.

US Patent 4,588,717 discloses fatty acid esters of phytosterol as vitamin supplements or as diet pills, said esters being made from a phytosterol and a C₁₈-C₂₀ fatty acid. Included as such fatty acids are also the unsaturated acids linolenic, linoleic and arachidonic acid. It is generally known that these acids have almost no effect on the levels of triglycerides.

WO 97/42830 discloses the manufacture and the use of gels consisting of partly crystallised mixtures of natural food oils with low concentrations of sterols and sterol esters ( especially sitosterol and oryzanol ), and optionally monoglycerides, in defined ratios as a means to give firmness to edible liquid fats. Because of the low sterol and sterol ester content, such products of necessity require substantial volumes and additional caloric content to deliver phytosterols and phytosterol esters in amounts to effectively lower cholesterol.

WO 98/06405 discloses a method of reducing cholesterol in the bloodstream by administering beta-sitostanol with campestanol in defined ratios as fatty acid esters derived from vegetable oils.

US Patent 5,502,045 describes reduction of cholesterol absorbtion into the bloodstream by administering beta-sitostanol esters of C₂-C₂₂ acids derived from vegetable oils.

In Journal of Lipid Research, 1993, 34, 1535-1544, there are described and referenced experiments with human subjects fed mixtures of sitostanol esters made from rapeseed oil fatty acids. The phytostanol esters were found to reduce serum LDL cholesterol more effectively than free phytosterols despite being hydrolyzed during intestinal passage.

In the European Journal of Clinical Nutrition, 1998, 52, 334-343 are presented results of human trials with margarines enriched with phytosterols and phytosterol esters. Plasma total and LDL cholesterol concentrations were shown to be reduced by sterol esters mixed with the margarines compared to controls with similar fatty acid profiles. All materials contained unsaturated fatty acid esters, especially those from oleic, linoleic or linolenic acid. No effect was seen on plasma triglyceride concentration with the sterol enriched margarines.

It has now been found that phytosterol and/or phytostanol esters made from phytosterols and/or phytostanols with certain omega-3 polyunsaturated fatty acids (n-3 fatty acids) are surprisingly effective in reducing both serum cholesterol and triglycerides. Such polyunsaturated fatty acids are, for example, eicosapentaenoic acid (EPA) having five unsaturated carbon-carbon double bonds or docosahexaenoic acid (DHA) with six unsaturated carbon-carbon double bonds. Said esters significantly lower both plasma cholesterol and triglyceride levels while phytosterol combined with vegetable oil only lower plasma cholesterol levels. Accordingly, these esters may be used as a combined cholesterol reduction agent and triglyceride lowering agent and, thus, positively affect two of the major risk factors for cardiovascular disease.

The above discussed effects have been shown in rats and the methods used and results obtained are described and summarized below.

### Animal treatment

Thirty male Fisher rats, weighing 177± 14 g, were maintained on a high fat diet (Table 1) during the 2 weeks preceding treatment. They were then randomly divided into five experimental groups consisting of 6 animals each. The control group (Group 1) remained on the high fat diet used during the 2 weeks pretreatment period. For the other experimental diets, in order to have isocaloric diets and an equal amount of fat in all the experimental diets, 2% (wt/wt) of the fat content of the control diet (1% coconut oil and 1% corn oil) was replaced by 2 % (wt/wt) of the following lipids:

| | |
|---|---|
| Group 2: | 2% sitosterol mix / high oleic sunflower oil (TRISUN 80) (1:1 ratio); |
| Group 3: | 2% sitostanol-DHA ester; |
| Group 4: | 2% stigmasterol-EPA ester; |
| Group 5: | 2% sitosterol mix + EPA/DHA ester (1:1 ratio)). |

The fatty acid compositions of the experimental diets are shown in Table 2. The rats were allowed free access to water and diet, and they were maintained on a 12-hour light-dark cycle. The diet in the cages was replaced daily, all unconsumed material discarded and food intake measured. Blood samples (1 ml) were taken by retroorbital puncture at the start of the experimental period (week 0) and after 2 weeks of treatment (week 2). After 4 weeks the animals were sacrificed by withdrawing blood from the vena Cava under Isoflurane anesthesia. Blood was collected into tubes containing EDTA as an anticoagulant.

### Lipid analysis

Plasma was prepared from the heparinized blood by immediate centrifugation at 1600 g for 10 minutes at 4°C. Assays of plasma cholesterol, triglycerides and HDL-cholesterol (precipitation method) were determined enzymatically on a COBASFARA analyzer (Roche Diagnostica, Switzerland). Non-HDL cholesterol was calculated by difference. The fatty acid composition of the diets was analyzed by gas chromatography.

### Statistical analysis

All data are expressed as means ± SD for animals in each diet group. The mean differences between dietary groups were analyzed by one-way analysis of covariance (ANCOVA) with subsequent Dunnet's test for multiple comparison against a control group (Group 1 and/or Group 2. The covariate adjusted for was the value of the corresponding parameter at the start of the treatment period (week 0). All tests were performed at the 5%-level and 95%-confidence intervals were calculated.

### Results

The growth of rats was similar in all dietary groups during the 4 weeks feeding period. The average food intake for the 4 weeks period of the five dietary regimes was 12 g/day/rat. Dietary treatment had no significant effect on body weight and food consumption.

The plasma cholesterol was significantly lower by 28% to 46% in all the four groups treated with phytosterols relative to control and by 46% to 66% relative to the pretreatment period (week 0) (Table 3).

The HDL cholesterol levels were almost not affected by the treatment with phytosterols (Table 4). Therefore, the non-HDL cholesterol (VLDL-Cholesterol + LDL cholesterol) were mainly lowered by phytosterol treament.

The plasma triglyceride levels were significantly lowered by 18% to 39% in the groups treated with phytosterol combined with n-3 fatty acids relative to the contro group and by 15% to 41% relative to the pretreatment period (week 0) (Table 5), whereas phytosterol combined with vegetable oil (Group 2) did not significantly lower plasma triglyceride.

**Table 1**

| Composition of the rat high fat diet^{a} | |
|---|---|
| Ingredients | g/100g anhydrous mix |
| Protein | 18.7 |
| Fiber | 6.6 |
| Fat | 18.3 |
| Carbohydrate | 39.2 |
| Dietary energy (MJ/Kg) | 16 |
| Metabolic energy in fat (%) | 42 |

The diet contained 0.5 wt% cholesterol, 1 wt% sodium cholate and the standard vitamin and mineral mix according to the requirements for rats

^{a}The main fats consisted of coconut kernel (18 wt%), coconut oil (2.5 wt%) and corn oil (2.5 wt%).

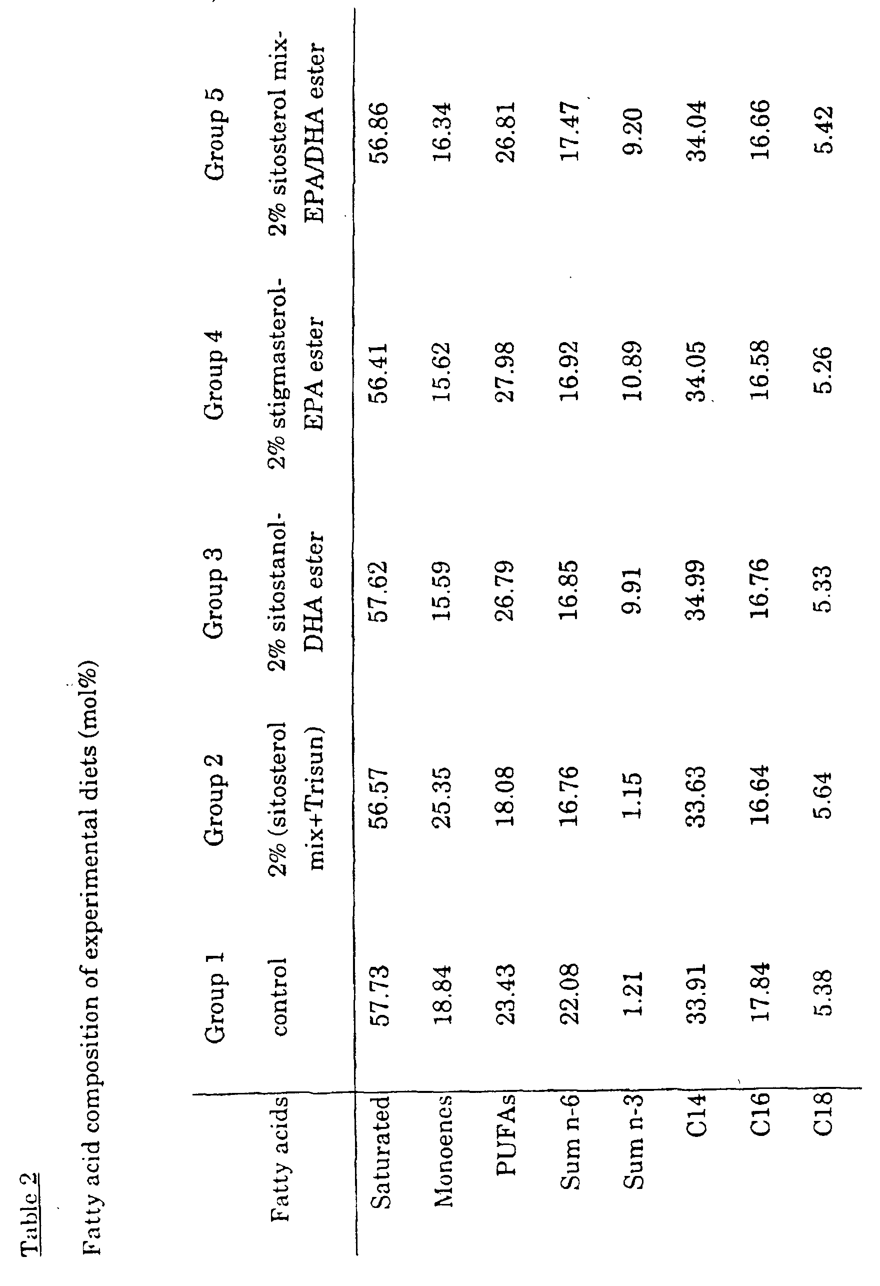

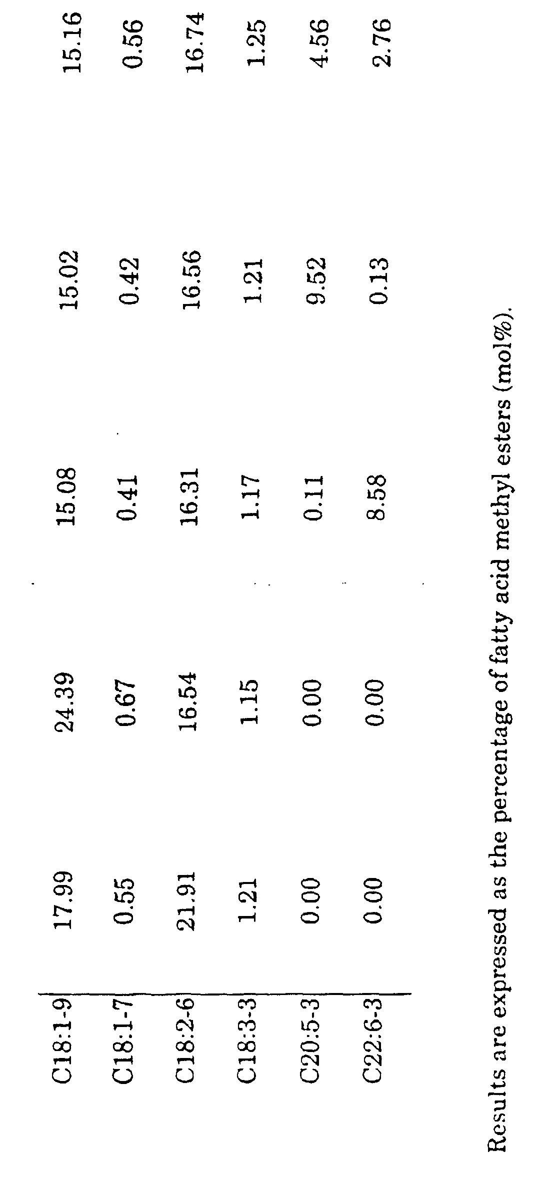

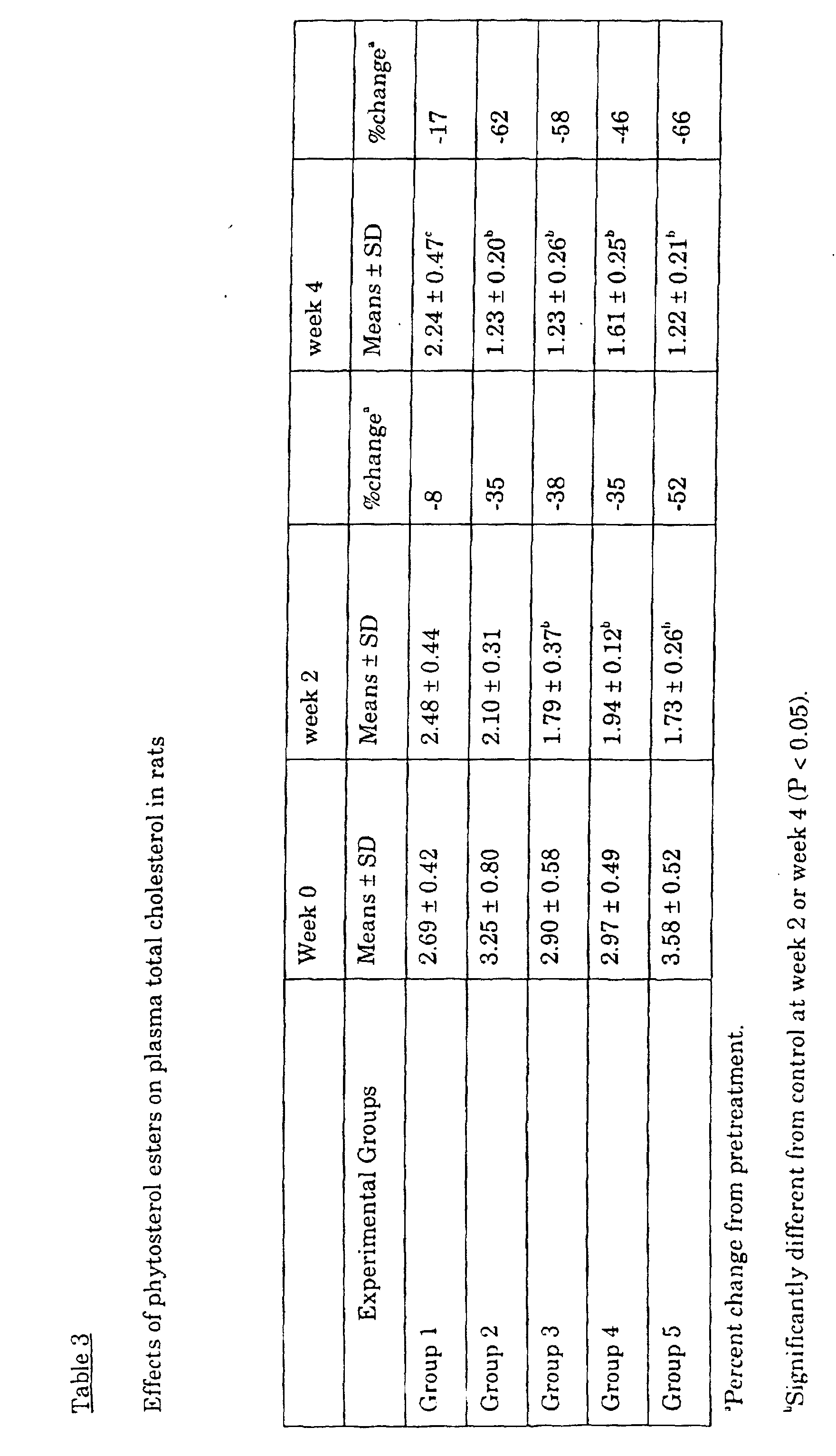

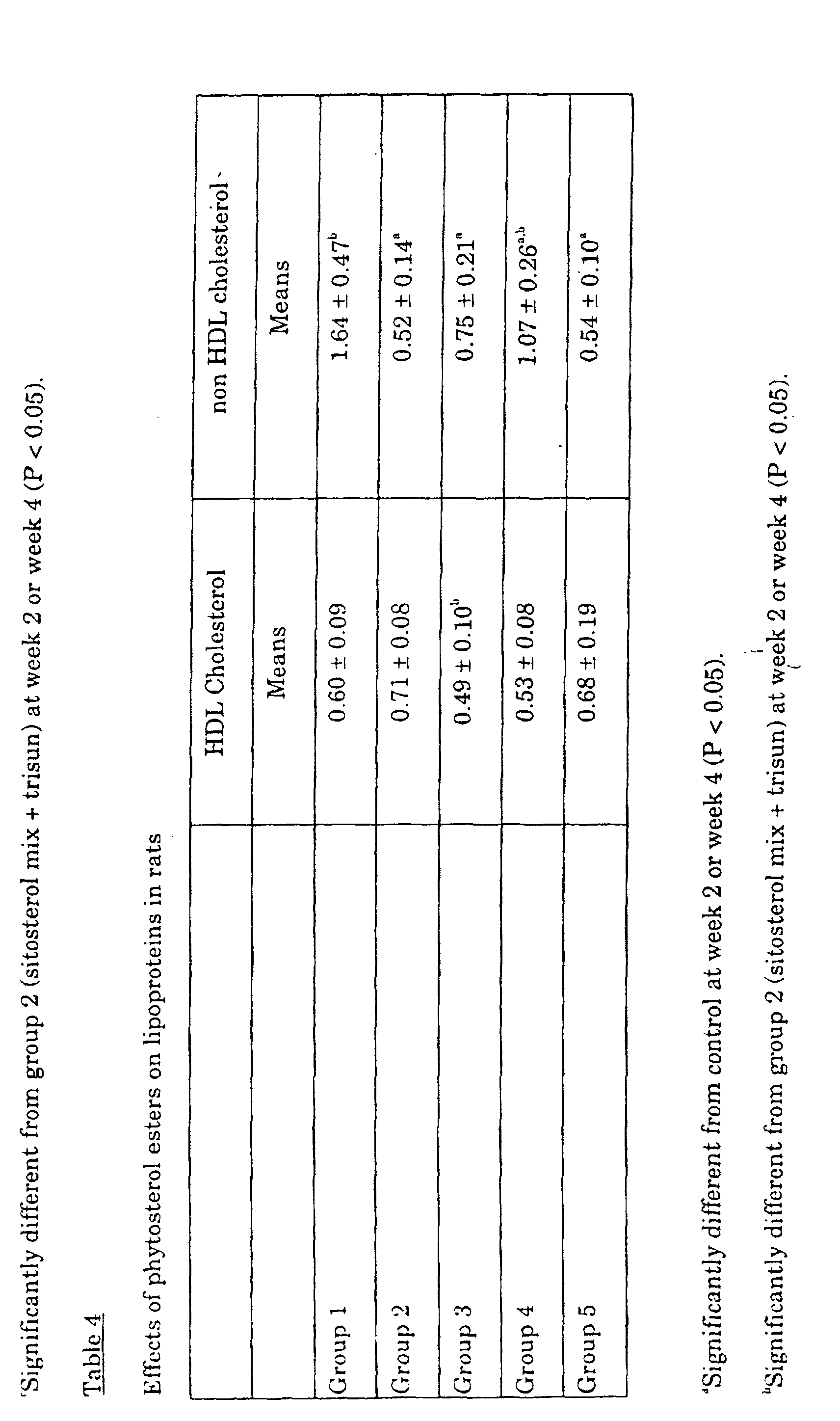

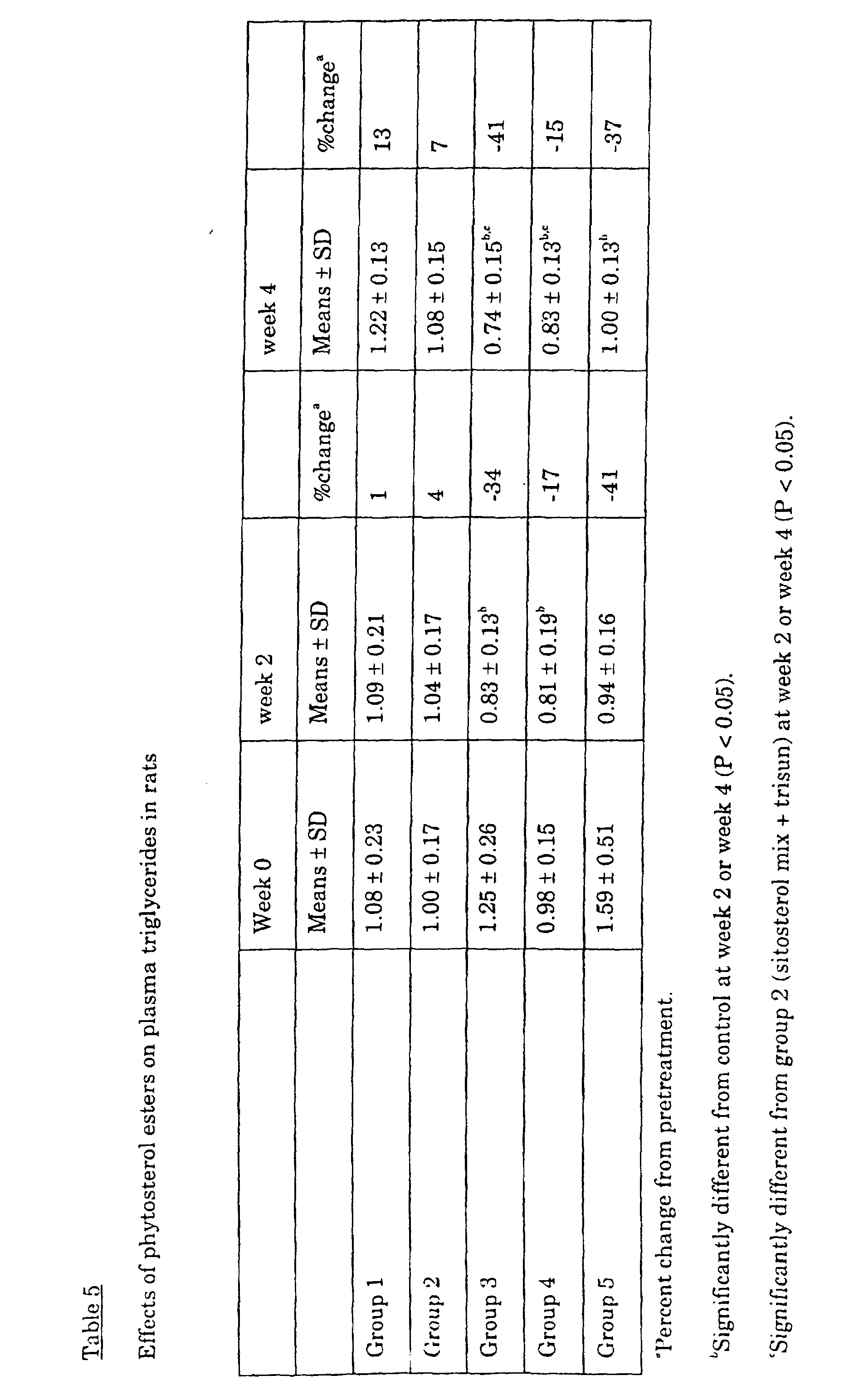

Physical properties of organic compounds such as physical state, melting point and solubility cannot be predicted reliably from chemical structures. These same properties contribute significantly to the acceptability of a food product by affecting texture, mouth feel or taste in complex and unpredictable ways. In the framework of the present invention there were synthesized esters of EPA and DHA with sitosterol, sitostanol and stigmasterol in pure form as well from mixtures of these sterols with other sterols and with mixtures of said acids with other fatty acids. Some of the compounds and mixtures were liquids whereas others were partly solid at room temperature or below. All were significantly more soluble in food oil than the corresponding phytosterols or phytostanols. For comparison there were also synthesized esters of sitostanol with mixed fatty acids containing significant levels of C₁₆-C₂₀ unsaturated fatty acids, especially linolenic acid, as obtained from rapeseed, and it was found that the mixtures produced were largely crystalline at room temperature and below. Much more food oil was required to completely dissolve these esters compared to the esters prepared with EPA or DHA.

It was further found that the compounds according to the present invention offer unique physical advantages. The compounds offer a higher solubility in food oils than other phytosterol esters so far described, which is of advantage for the incorporation into a variety of food products. These materials allow co-delivery of phytosterols and/or phytostanols and selected PUFAs in their ester form in the highest concentration per unit volume possible. This is of advantage for incorporation into products where smaller volumes are important, such as in water dispersible formulations, or where additional non-essential food oils are undesirable. This presents physical advantages over simple mixtures or formulations of other phytosterols/phytostanols and/or their fatty esters with PUFAs and their normally available ester or triglyceride forms.

Accordingly, an object of the present invention is the use of phytosterol and/or phytostanol esters with PUFAs having from 18 to 22 carbon atoms and at least three unsaturated carbon-carbon double bonds for the preparation of formulations, in suitable physical forms, such as in capsules etc., as diet supplements or as ingredients in foods for the purpose of lowering serum cholesterol levels and serum triglyceride levels in humans. The compounds according to the present invention are used preferably at total amounts of 1 to 10 grams per day of phytosterol ester and/or phytostanol ester content.

Preferred phytosterols are beta-sitosterol or stigmasterol and campesterol or mixtures thereof. More preferred are beta-sitosterol and stigmasterol or mixtures thereof. Most preferred is beta-sitosterol. Preferred phytostanols are beta-sitostanol and campestanol or mixtures thereof. More preferred is beta-sitostanol. Preferred PUFAs are EPA and DHA

It is readily understood that the esters of the present invention need not be used in a pure state. Mixture of these esters may be used. Likewise mixtures of these esters with other fatty esters of phytosterols/phytostanols may be used. The ratios of phytosterol and/or phytostanols used may vary with their source. Likewise the ratios of PUFA and other fatty acids may vary. It is also understood that the products may contain some free phytosterols/phytostanols and/or PUFA glycerides or esters. The physical properties can as a consequence be varied from those with a high proportion of polyunsaturated phytosterol/stanol esters which are liquids well soluble in food oils to those of the mixture with lesser proportions of unsaturation which are semisolid or waxy.

The compounds according to the present invention can be prepared according to known methods. For example they can be obtained by esterifying a phytosterol/phytostanol with a n-3 PUFA in a known manner. Alternatively, they can preferably be prepared by interesterification of free phytosterols/ phytostanols with esters of the n-3 PUFAs by heating in the presence of an interesterification catalyst, whereby (i) the interesterification is carried out solvent free, (ii) the fatty esters include suitable simple C₁-C₄-esters and triglycerides, (iii) the catalyst is a sodium alkoxide of a C₁-C₄-alcohol. The reaction is suitably conducted by heating the mixture at 80-140°C at a pressure of 133-6650 Pa whereby the reaction preferably is carried out with a stoichiometric amount to an excess of the PUFA ester. The following examples further illustrate the invention.

### Example 1

To a mixture of 0.91 g of docosahexaenoic acid (purity: 90%), 1.03 g of stigmasterol (purity: 95%) and dimethylaminopyridine (50 mg) in 18 ml of dry dichloromethane was added a solution of dicyclohexylcarbodiimide (0.63 g) in 5 ml dichloromethane. After 4 hours stirring at room temperature, the reaction was complete. Then, methanol (0.5 g) and acetic acid (0.25 g) were added and the mixture stirred for a further one hour. The mixture was cooled to 0°C, filtered, and the solids rinsed with hexane (3 x 25 ml). The solvent was removed under reduced pressure and the residue flash chromatographed on silica to yield a pure fraction of 1.0 g of stigmasterol docosahexaenoate as a colourless oil with consistent NMR and IR data. This substance remained an oil when stored for several weeks at room temperature and when cooled for several weeks at -20°C.

### Example 2

Analogous to Example 1, stigmasterol eicosapenatenoate was prepared from eicosapentaenoic acid (purity: 90%) and stigmasterol. Stigmasterol eicosapenatenoate (1.46 g) was obtained as a colourless oil which remained liquid within a temperature range of 20°C and -20°C.

### Example 3

Analogous to Example 1 a mixture of eicosapentaenoic acid-docosahexaenoic acid esters of stigmasterol was prepared from stigmasterol with a mixture of 49% eicosapentaenoic acid and 27% docosahexaenoic acid. The mixture of the esters of stigmasterol was obtained as a colourless oil which remained liquid within a temperature range of 20°C and -20°C.

### Example 4

Analogous to Example 1 stigmastanol docosahexaenoate was prepared from stigmastanol (purity: 95%) and docosahexaenoic acid (purity: 90%). Stigmastanol docosahexaenoate was obtained as a slightly coloured oil which remained liquid between 20°C and -20°C.

### Example 5

Analogous to Example 1 stigmastanol eicosapentenoate was prepared from stigmastanol and eicosapentaenoic acid. Stigmastanol eicosapentenoate was obtained as a slightly yellowish oil which remained liquid within the temperature range of 20°C and -20°C.

### Example 6

Analogous to Example 1 a mixture of stigmastanol eicosapentaenoic acid and docosahexaenoic acid esters was prepared from stigmastanol and a mixture of 49% eicosapentaenoic acid with 27% docosahexaenoic acid. A mixture of stigmastanol eicosapentaenoic acid and docosahexaenoic acid esters was obtained as a colourless oil which became turbid when stored at 20°C and partly solid at -20°C.

### Example 7

Analogous to Example 1 a mixture of sterol PUFA esters was prepared from a mixture of beta-sitosterol, campesterol, and stigmasterol and a mixture of 49% eicosapentaenoic acid with 27% docosahexaenoic acid. A mixture of sterol PUFA-esters was obtained as a turbid oil containing some solids at 20°C and partly solid at -20°C.

### Example 8

Analogous to Example 1, a mixture of stigmastanol unsaturated fatty esters was prepared from stigmastanol and a mixture of fatty acids obtained from basic hydrolysis of a commercial food sample of Swiss rapeseed oil (9% saturated, 61% monounsaturated, 30% polyunsaturated triglycerides). A mixture of stigmastanol unsaturated fatty esters was obtained as a colourless oil which slowly crystallised at room temperature. At -20 C the material was essentially solid.

### Example 9

A mixture of phytosterols (20.6 g of a commercial mixture of sitosterol 43%, stigmasterol 23%, campesterol 24% with other minor sterols) and 75% DHA-EPA ethyl esters (16.8 g of a commercial mixture of 43% ethyl docosahexaenoate and 32% ethyl eicosapentaenoate with other fatty esters) was dried at 120°C while sparging with a stream of inert gas. To the molten mixture was added sodium ethoxide (1.03 ml 21% solution in ethanol). The mixture was stirred at 120°C at 15 mbar vacuum for two hours. The light brown mixture was cooled to 80°C and the catalyst quenched with dilute acid. The separated oil phase was dehydrated by heating under reduced pressure while sparging with a stream of inert gas. Obtained were 35.0 g of crude phytosterol esters as a turbid light brown oil, which remained fluid at room temperature. HPLC showed that the conversion to sterol esters was 95%.

### Example 10

A mixture of phytosterols (148 g of a commercial mixture of sitosterol 43%, stigmasterol 23%, campesterol 24% with other minor sterols) and fishoil glycerides (141 g of a commercial mixture of glycerides with fatty acid composition of 17% EPA and 11% DHA) was dehydrated by sparging at 120°C with inert gas. To the molten mixture was added sodium ethoxide (11.9 ml of 21% solution in ethanol). The mixture was stirred at 120°C at 15 mbar vacuum for one hour.

The light brown mixture was quenched with dilute acid, and the separated oil phase dehydrated under reduced pressure to afford 249 g of a light brown oil which slowly crystallized to a semisolid mass. HPLC showed that the conversion was 93%.

### Example 11

Solubilities of materials made according to the procedures described in Examples 1-8, as well as the parent sterols were assessed in a commercial sample of Swiss rapeseed oil by alternately adding small increments of oil at room temperature to weighed amounts of sterol esters and agitating for 5 minute periods until solution was attained. Minimum starting ratio was about 1:1 and trials were discontinued at above 10:1.

| material | solubility g oil /g material |
|---|---|
| stigmasterol docosahexaenoate | miscible > 1 |
| stigmasterol eicosapentenoate | miscible > 1 |
| stigmasterol EPA-DHA ester mixture | miscible > 1 |
| stigmastanol docosahexaenoate | miscible > 1 |
| stigmastanol eicosapentaneoate | miscible > 1 |
| stigmastanol EPA-DHA ester mixture | soluble > 4 |
| sitosterol sterols mix EPA-DHA ester mixture | miscible > 1 |
| stigmastanol rape-seed ester mixture | insoluble > 10 |
| stigmasterol | insoluble > 10 |
| stigmastanol | insoluble > 10 |
| docosahexaenoic acid ethyl ester 90% | miscible > 1 |
| EPA ethyl ester 90% | miscible > 1 |

## Claims

1. Use of phytosterol and/or phytostanol esters with polyunsaturated fatty acids having from 18 to 22 carbon atoms and at least three unsaturated carbon-carbon double bonds and mixtures thereof for the preparation of formulations, suitable physical forms, diet supplements or food ingredients for the purpose of lowering serum cholesterol and simultaneously lowering serum triglycerides.

2. The use of esters according to claim 1, wherein the phytosterol is beta-sitosterol, stigmasterol or campesterol or a mixture thereof, preferably beta-sitosterol or stigmasterol or a mixture thereof, most preferably beta-sitosterol.

3. The use of esters according to claim 1 or 2, wherein the phytostanol is campestanol or beta-sitostanol or a mixture thereof, preferably beta-sitostanol.

4. The use of esters according to any one of claims 1 to 3, wherein the polyunsaturated fatty acid is eicosapentaenoic acid or docosahexaenoic acid.

5. The use of esters according to any one of claims 1 to 4, wherein the esters further comprise admixtures of esters of phytosterol and/or phytostanol with fatty acids other than those polyunsaturated fatty acids specified in claim 1 or claim 4, and/or which further comprise admixtures of free phytosterols/ phytostanols and/or PUFA glycerides or esters.

6. The use of esters according to claim 1, wherein the suitable physical form is a capsule.

## Patentansprüche

1. Verwendung von Phytosterol- und/oder Phytostanolestern mit mehrfach ungesättigten Fettsäuren mit 18 bis 22 Kohlenstoffatomen und mindestens drei ungesättigten Kohlenstoff-Kohlenstoff-Doppelbindungen und Mischungen davon zur Herstellung von Präparaten, geeigneten physikalischen Formen, Nahrungsergänzungen oder Lebensmittelinhaltsstoffen mit dem Zweck, Serumcholesterin und gleichzeitig Serumtriglyceride zu senken.

2. Verwendung von Estern nach Anspruch 1, wobei das Phytosterol β-Sitosterol, Stigmasterol oder Campesterol oder eine Mischung davon, bevorzugt β-Sitosterol oder Stigmasterol oder eine Mischung davon, am meisten bevorzugt β-Sitosterol ist.

3. Verwendung von Estern nach Anspruch 1 oder Anspruch 2, wobei das Phytostanol Campestanol oder β-Sitostanol oder eine Mischung davon, bevorzugt β-Sitostanol ist.

4. Verwendung von Estern nach einem der Ansprüche 1 bis 3, wobei die mehrfach ungesättigte Fettsäure Eicosapentaensäure oder Docosahexaensäure ist.

5. Verwendung von Estern nach einem der Ansprüche 1 bis 4, wobei die Ester weiterhin Mischungen von Estern von Phytosterol und/oder Phytostanol mit anderen Fettsäuren als den mehrfach ungesättigten Fettsäuren, die in Anspruch 1 oder Anspruch 4 angegeben sind, aufweisen und/oder weiterhin Mischungen von freien Phytosterolen/Phytostanolen und/oder PUFA-Glyceriden oder Estern enthalten.

6. Verwendung von Estern nach Anspruch 1, wobei die geeignete physikalische Form eine Kapsel ist.

## Revendications

1. Utilisation d'esters de phytostérol et/ou phytostanol avec des acides gras polyinsaturés ayant de 18 à 22 atomes de carbone et comportant au moins trois doubles liaisons carbone-carbone insaturées, et de mélanges de ceux-ci, pour la préparation de compositions, formes physiques convenables, compléments nutritionnels ou ingrédients alimentaires dans le but d'abaisser la cholestérolémie et en même temps d'abaisser le taux de triglycérides sériques.

2. Utilisation d'esters selon la revendication 1, dans laquelle le phytostérol est le β-sitostérol, le stigmastérol ou le campestrol ou un mélange de ceux-ci, de préférence le β-sitostérol ou le stigmasétrol ou un mélange de ceux-ci, de façon particulièrement préférée le β-sitostérol.

3. Utilisation d'esters selon la revendication 1 ou 2, dans laquelle le phytostérol est le campestanol ou le β-sitostanol ou un mélange de ceux-ci, de préférence le β-sitostanol.

4. Utilisation d'esters selon l'une quelconque des revendications 1 à 3, dans laquelle l'acide gras polyinsaturé est l'acide eicosapenténoïque ou l'acide docosahexaénoïque.

5. Utilisation d'esters selon l'une quelconque des revendications 1 à 4, dans laquelle les esters en outre comprennent des mélanges d'esters de phytostérol et/ou phytostanol avec des acides gras autres que les acides gras polyinsaturés spécifiés dans la revendication 1 ou la revendication 4, et/ou qui en outre comprennent des mélanges de phytostérols/phytostanols libres et/ou de glycérides ou d'esters d'AGPU (acides gras polyinsaturés).

6. Utilisation d'esters selon la revendication 1, dans laquelle la forme physique est une gélule.
